# EUROPEAN PATENT SPECIFICATION

(11) **EP 1 993 996 B1**
(45) Date of publication and mention of the grant of the patent: **14.09.2011**
(21) Application number: 07758055.3
(22) Date of filing: 07.03.2007
(51) Int. Cl.: C07C 253/30

(54) **A METHOD FOR PRODUCING A CYANOACRYLATE MONOMER**
VERFAHREN ZUR HERSTELLUNG EINES CYANOACRYLATMONOMERS
PROCEDE DE PRODUCTION D'UN MONOMERE CYANOACRYLATE

(30) Priority: 10.03.2006 US 372959
(43) Date of publication of application: 26.11.2008
(73) Proprietor: Ethicon, Inc, Somerville, NJ 08876-0151 (US)
(72) Inventor: LIU, Hongbo, Hillsborough, New Jersey 08844 (US); GONZALEZ, Sandra, Bedminster, New Jersey 07921 (US)
(74) Representative: James, Anthony Christopher W.P.
(86) International application number: PCT/US2007/063467
(87) International publication number: WO 2007/106688

(56) References cited:
- EP-A1- 0 470 722
- WO-A2-2006/120628
- US-A- 2 912 454
- US-A- 4 328 170
- US-A1- 2002 141 969
- YU-HAUR HWANG, CHI-PING HWANG, RAYMOND CHIEN-CHAO TSIANG, MICHAEL CHEN: "Synthesis and Characterisation of Ethoxyethyl alpha-Cyanoacrylate and Reaction Intermediates" JOURNAL OF APPLIED POLYMER SCIENCE, vol. 87, 2003, pages 1758-1773, XP002441308
- KONSTANTINOV, C. ET AL.: "über Synthese und Eigenschaften von Ethoxyethyl- und n-Hexyl-alpha-cyanacrylatklebstoffen" PLASTE UND KAUTSCHUK, vol. 12, 1981, pages 697-698, XP008080984
- V.VIJAYALAKSHMI ET AL: J. ADHESION SCI. TECHNOL., vol. 4, no. 9, 1990, pages 733-750, XP008080838 cited in the application

## Description

### Field of the Invention

A method for producing cyanoacrylate monomers, which may be polymerized to form an adhesive, is described herein.

### Background

Monomer and polymer adhesives are used in both industrial (including household) and medical applications. Included among these adhesives are the cyanoacrylate monomers and polymers, such as the α- cyanoacrylates. Since the discovery of the adhesive properties of these monomers and polymers, they have found wide use due to the speed with which they cure, the strength of the resulting bond formed, and their relative ease of use. These characteristics have made the α- cyanoacrylate adhesives the primary choice for numerous applications such as bonding plastics, rubbers, glass, metals, wood, and more recently, biological tissues.

Cyanoacrylate monomers are generally produced by forming α-cyanoacrylate prepolymer or oligomer and then cracking or depolymerizing the α-cyanoacrylate prepolymer or oligomer to produce monomeric cyanoacrylate. More particularly, α-cyanoacrylate prepolymer or oligomer is commonly produced by first reacting cyanoacetate with formaldehyde, or a functional equivalent such as the polymeric form paraformaldehyde, in the presence of a base, which acts as a catalyst for the reaction between the cyanoacetate and paraformaldehyde to produce the α-cyanoacrylate prepolymer or oligomer. Depolymerization is generally effected by heating the polymer under reduced pressure while distilling off and condensing the monomer as it is being generated.

Monomers of α-cyanoacrylates are anionically polymerizable or free radical polymerizable, or polymerizable by zwitterions or ion pairs to form adhesive polymers. Once polymerization has been initiated, the cure rate can be very rapid. It is also known that monomeric forms of α-cyanoacrylates are extremely reactive, and that polymerization can be inititiated very easily in the presence of even minute amounts of an initiator, such as moisture present in the air or on moist surfaces such as animal (including human) tissue. Although these are desirable properties of the cyanoacrylate monomers, it is desirable to strike a balance between maintaining the cyanoacrylate monomers in its monomeric form, inititiating polymerization at a time when polymerization is desirable to form an adhesive bond, without reducing the cure rate of the adhesive.

Due to the inherent high reactivity of the monomeric α-cyanocrylates, *in situ* polymerization or re-polymerization often occurs during the depolymerization step of the manufacturing process, preventing effective recovery of the monomer. This in situ polymerization or re-polymerization problem is particularly severe for highly reactive monomers such as short chain alkyl cyanoacrylates and cyanoacrylate with side chain ester groups.

USP2,721,858 and USP6,057,472 report the use of acidic gaseous inhibitors to maintain the cyanoacrylate monomers in their monomeric form, i.e., to prevent premature initiation and/or polymerization of the monomers. In particular, gaseous acidic inhibitors such as sulfur dioxide, nitric oxide, hydrogen fluoride, and the like, are combined with the monomers as they are formed during the depolymerization process. More specifically, the gaseous acidic inhibitors mix with the monomeric vapors as they are produced during the depolymerization process and dissolve in the monomeric liquid when the monomeric vapors are collected and condensed into a liquid. The presence of the acidic inhibitors in the monomeric vapors and condensed liquid prevent the monomers from prematurely polymerizing during the manufacturing process. However, the use of the gaseous acidic inhibitors described in the prior art requires additional operations for feeding and maintaining a steady stream of inhibitors, as well as for disposing these harmful gases at the end of the process. Furthermore, there is a potential for an excess amount of inhibitors to be introduced into the monomers produced, which may adversely affect the initiation of the polymerization of the monomer and the cure rate of the adhesive.

Konstantinov et al (Plaste und Kautschuk, 12, 697-698 (1981)) disclose the synthesis of ethoxyethyl and n-hexyl- α- cyanoacrylate from the corresponding cyanoacetate by reaction with the paraformaldehyde in the presence of piperidine hydroquinone and phosphorus pentoxide are used in the depolimerization.

V. Vijayalakshmi, et al. (Alkyl and Substituted Alkyl 2-Cyanoacrylates. Part 1. Synthesis and Properties, V. Vijayalakshmi, et al., J. Adhesion Sci. Technol., 4(9), 733-750 (1990)), report the use of phosphoric acid to neutralize the base catalysts used to prepare the cyanoacrylate prepolymer or oligomer after the formation of the cyanoacrylate prepolymer or oligomer. The solid salts formed by the neutralization reaction are removed by decanting the aliquot. The cyanoacrylate prepolymer or oligomer is then depolymerized by heat in the presence of P₂O₅ and hydroquinone. The authors report the successful synthesis of a range of cyanoacrylate monomers. However, it is believed that relatively non-volatile phosphoric acid remains in the prepolymer or oligomer, which could potentially promote undesired side reactions, i.e., such as hydrolysis, during the depolymerization process. For the preparation of cyanoacrylate monomers, such as alkoxycarboalkyl cyanoacrylates, that are susceptible to hydrolytic decomposition reactions, such potentially undesirable reactions may result in reduced yields and an increased amount and complexity of impurities in the final monomeric product

Although various efforts have been reported in the prior art to improve the depolymerization process, there is a need for a process that avoids the use of harmful gases such as sulfur dioxide, nitric oxide, hydrogen fluoride, or the use of neutralizing agents that have the potential to promote undesired side reactions, i.e., such as hydrolysis, during the depolymerization process.

### SUMMARY OF THE INVENTION

A method is described for producing a cyanoacrylate monomer, comprising the steps of: (a) reacting cyanoacetate and formaldehyde or paraformaldehyde in the presence of a base or amine base catalyst to form an α-cyanoacrylate prepolymer or oligomer mixture; (b) subjecting the α-cyanoacrylate prepolymer or oligomer mixture to a substrate to obtain a purified α-cyanoacrylate prepolymer or oligomer by removing the catalyst; wherein, either (i) the catalyst is a base catalyst other than an amine base catalyst and said substrate is an acidic ion exchange resin, or (ii) said catalyst is an amine base catalyst and said substrate is an acidic ion exchange resin or a resin bearing other amine reacting functional groups selected from anhydrides, acid halides, aldehydes, ketones and isocyanates; (c) subjecting the purified α-cyanoacrylate prepolymer or oligomer to depolymerization to obtain a α-cyanoacrylate monomer.

### DETAILED DESCRIPTION OF THE INVENTION

Cyanoacrylate monomers are produced in the conventional manner by first reacting or condensing cyanoacetate with formaldehyde, or a functional equivalent such as the polymeric form paraformaldehyde, in the presence of a base or amine base, which acts as a catalyst for the reaction between the cyanoacetate and paraformaldehyde to produce the α-cyanoacrylate prepolymer or oligomer.

The α-cyanoacrylate prepolymer or oligomer is then subjected to a substrate said substrate being an acidic ion exchange resin when the catalysts is a base catalyst other than an amine base catalyst or an acidic in exchange resin or a resin beavring other amine reacting functional groups sented from anhydrides, acid halids, aldehydes, tatone on isocyanates when the catyst is an amine used catalyst. to remove the catalyst and to obtain a purified α-cyanoacrylate prepolymer or oligomer according to the present invention. For example, the prepolymer or oligomer, in the form of a solution in an appropriate organic solvent, such as methylene chloride, acetone, ethylacetate, etc., may be exposed to resin to obtain a purified α- cyanoacrylate prepolymer or oligomer by removing the catalyst.

The catalyst used herein includes base catalyst and amine base catalyst. Base catalysts that may be used to condense the cyanoacetate with formaldehyde, or the functional equivalent such as the polymeric form paraformaldehyde, include but are not limited to alkali metal or alkaline earth hydroxide, such as sodium or potassium hydroxide, sodium or potassium methoxide and sodium or potassium t-butoxide; and carbonates or bicarbonates, such as sodium or potassium carbonate and sodium or potassium bicarbonate.

When the catalyst is a base catalyst, the resin may be an acidic ion-exchange resin. Specifically, the prepolymer or oligomer solution may be introduced into a packed column of the acidic ion-exchange resin or the prepolymer or oligomer solution may be put into a suspension of acidic ion-exchange resin particles. The acid groups present on the resin that may be used for removing a base catalyst include are but not limited to sulfonic acid and carboxylic acid. Examples of commercially available acidic ion-exchange resins include acidic Dowex^{®} macroporous resins from The Dow Chemical Company (Midland, Michigan, U.S.A.), macroporous sulfonic acid resins such as Product No. 8022-2, from Agela Technologies, Inc. (Newark, DE, USA), and Amberlite^{®} resins from Rohm & Haas (Philadelphia, Pennsylvania, USA).

Amine base catalysts that may be used include but are not limited to primary, secondary, and tertiary amines. Examples of amine base catalysts include but are not limited to alkylamines such as methylamine, dialkylamine such as dimethylamine, trialkylamines such as triethylamine, cyclic amines such as piperidine, aromatic amines such as pyridines, and hydroxylamines.

When the catalyst is an amine base catalyst, the acidic ion-exchange resins described above may be used, as well as resins bearing other amine reacting functional groups other than acid groups. Such amine reacting functional groups include but are not limited to acid derivatives such as anhydrides and acid halides, aldehydes, ketones, and isocyanates. Examples of commercially available resins bearing amine reacting functional groups are Stratosphere^{™} PL-FMP resin and HypoGel^{®} aldehyde resins (Sigma-Aldrich Corp., St. Louis, Missouri, USA).

The amount of resin required should be sufficient to substantially remove the catalyst. For example, the molar ratio of the reactive functional groups on the resin to the catalyst may range from about 1:1 to about 100:1; preferably from about 2:1 to about 50:1; and more preferably from about 5:1 to about 20:1.

Depolymerization is then effected in the conventional manner by heating the polymer under reduced pressure while distilling off and collecting the monomer as it is being generated.

Suitable cyanoacetates that may be utilized in the process described herein may be represented by the following formula (I):

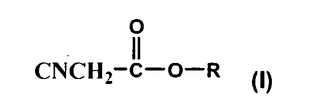

R may be an alkyl group, linear, branched or cyclic, having a combined number of carbon atoms from 1 to 20. Alternatively, R may be a group having a formula R₂-O-R₃, where R₂ and R₃ are each independently an alkyl group, linear, branched or cyclic, having a combined number of carbon atoms from 1 to 20; or R may be group having a formula R₄-COO-R₅, where R₄ and R₅ are each independently an alkyl group, linear, branched or cyclic, having a combined number of carbon atoms from 1 to 20. Examples of cyanoacetates that may be utilized in the process described herein include but are not limited to alkyl cyanoacetates, alkoxyalkyl cyanoacetates, alkoxycarboalkyl cyanoacetates and the combination thereof. Particular examples include but are not limited to the following cyanoacetates: 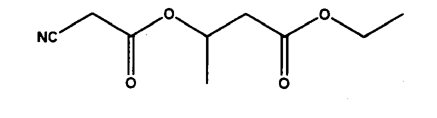 3-(2-Cyano-acetoxy)-butyric acid ethyl ester (Et-β-HBT-CAc) 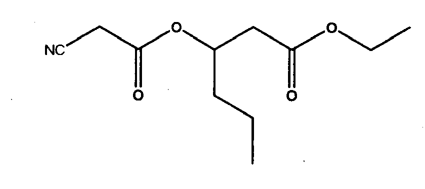 3-(2-Cyano-acetoxy)-hexanoic acid ethyl ester (Et-β-CPL-CAc)

Cyanoacrylates that may be prepared according to the methods described herein can be represented by the following formula (II):

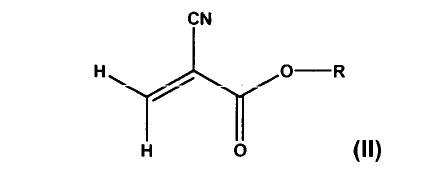

R may be an alkyl group, linear, branched or cyclic, having a combined number of carbon atoms from 1 to 20. Alternatively, R may be a group having a formula R₂-O-R₃, where R₂ and R₃ are each independently an alkyl group, linear, branched or cyclic, having a combined number of carbon atoms from 1 to 20; or R may be group having a formula R₄-COO-R₅, where R₄ and R₅ are each independently an alkyl group, linear, branched or cyclic, having a combined number of carbon atoms from 1 to 20. Examples of cyanoacrylates include but are not limited to alkyl cyanoacrylates, alkoxyalkyl cyanoacrylates, alkoxycarboalkyl cyanoacrylates and combination thereof. Particular examples include but are not limited to the following cyanoacrylates: 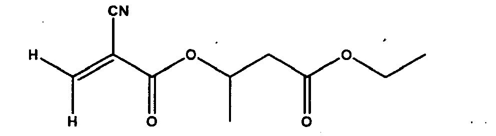 3-(2-Cyano-acryloyloxy)-butyric acid ethyl ester (Et-β-HBT-CA) 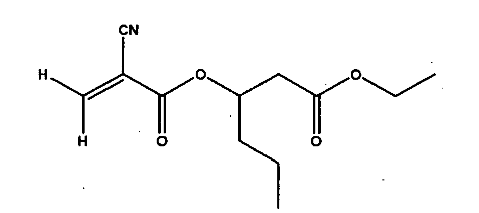 3-(2-Cyano-acryloyloxy)-hexanoic acid ethyl ester (Et-β-CPL-CA)

While the following examples demonstrate certain embodiments of the invention, they are not to be interpreted as limiting the scope of the invention, but rather as contributing to a complete description of the invention

### Comparative Example 1A

### Synthesis of 3-(2-Cyano-acryloyloxy)-hexanoic acid ethyl ester (Et-β-CPL-CA)

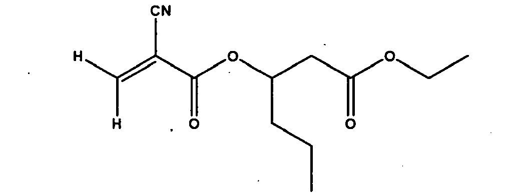

3-(2-Cyano-acryloyloxy)-hexanoic acid ethyl ester (Et-β-CPL-CA)

A mixture of 45.45g of 3-(2-Cyano-acetoxy)-hexanoic acid ethyl ester (Et-β-CPL-CAc), 7.2g of paraformaldehyde, 0.06ml of piperidine and 150ml toluene were stirred in a 250ml round bottom flask equipped with a magnetic stir bar. A Dean-Stark trap and a condenser were attached to the reaction flask. The reaction flask was immersed in an oil bath. The reaction mixture was heated to reflux and the reaction was allowed to proceed overnight.

The slightly brown colored reaction mixture was evaporated using a rotary evaporator to remove the solvent, yielding a brown colored viscous residue which turned into a solid gel after cooling to room temperature. This solid gel was oligomer of 3-(2-Cyano-acryloyloxy)-hexanoic acid ethyl ester.

The oligomer, 0.40g of hydroquinone (HQ) and 2.0g of P₂O₅ were combined in a 250ml round bottom flask. A simple vacuum distillation was set up where all glassware pieces were previously treated with 5N H₂SO solution and dried in a vacuum oven after rinsing with deionized water (Dl water). The above mixture of oligomer, hydroquinone and P₂O₅ was heated to up to 140°C to remove low boiling impurities and then to above 160°C in an oil bath under vacuum to carry out the depolymerization. The monomeric Et-β-CPL-CA was generated from the reaction but rapidly polymerized along the distillation path. No free flowing monomeric Et-β-CPL-CA was collected in the receiving end. Instead, 6.7g of polymerized mass was recovered as a colorless and transparent solid.

### Example 1B

### Synthesis of 3-(2-Cyano-acryloyloxy)-hexanoic acid ethyl ester (Et-β-CPL-CA)

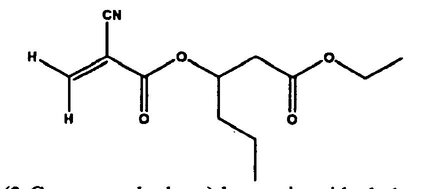

3-(2-Cyano-acryloyloxy)-hexanoic acid ethyl ester (Et-β-CPL-CA)

A mixture of 45.45g of 3-(2-Cyano-acetoxy)-hexanoic acid ethyl ester (Et-β-CPL-CAc), 7.2g of paraformaldehyde, 0.06ml of piperidine and 150ml toluene was stirred in a 250ml round bottom flask equipped with a magnetic stir bar. A Dean-Stark trap and a condenser were attached to the reaction flask. The reaction flask was immersed in an oil bath. The reaction mixture was heated to reflux and the reaction was allowed to proceed overnight.

The slightly brown colored reaction mixture was evaporated using a rotary evaporator to remove the solvent, yielding a brown colored viscous residue which turned into a solid gel after cooling to room temperature. This solid gel was oligomer of 3-(2-Cyano-acryloyloxy)-hexanoic acid ethyl ester.

A macroporous sulfonic acid ion-exchange resin (MP-SO₃H resin, Product No. 8022-2, Agela Technologies, Inc., Newark, Delaware, USA) in the amount of 18g was conditioned in 150ml CH₂Cl₂ for one hour. The conditioned resin was poured into a glass column (with sintering filter at the bottom) with 3·18cm O.D to form a tightly packed column with a height of about 9·40cm On top of this packed column a layer of sand was deposited.

The oligomer of 3-(2-Cyano-acryloyloxy)-hexanoic acid ethyl ester was dissolved in 150ml CH₂Cl₂ to form a brownish solution. This solution was eluted through the above packed column by gravity. Slightly yellow colored oligomer solution was collected and evaporated to obtain a slightly yellow colored solid gel. This solid gel was depolymerized as described in Example 1A. Two separate monomeric 3-(2-Cyano-acryloyloxy)-hexanoic acid ethyl ester fractions were collected from the depolymerization reaction, an early fraction of 21g (88% by GC) and a later fraction of 7g (99% by GC). Both fractions were free flowing colorless liquids. No polymerization along the distillation path was observed as in Example 1 A. The less pure early fraction was subjected to a second distillation under vacuum yielding 13g of monomeric 3-(2-Cyano-acryloyloxy)-hexanoic acid ethyl ester with improved purity (97% by GC). A combined 20% yield of the monomeric 3-(2-Cyano-acryloyloxy)-hexanoic acid ethyl ester was obtained as a result.

A very small amount of this monomer product was placed in between two moist fingertips and bonded the fingertips strongly within one minute.
¹H NMR (CDCl₃, δppm): 7.03(s, 1H), 6.60(s, 1H), 5.40(m, 1H), 4.15(q, 2H), 2.65(m, 2H), 1.70(m, 2H), 1.38(m, 2H), 1.22(t, 3H), 0.95(t, 3H) Boiling point: 107-114°C 53·33 Pa

### Example 2.

### Synthesis of 3-(2-Cyano-acryloyloxy)-butyric acid ethyl ester (Et-β-HBT-CA)

3-(2-Cyano-acryloyloxy)-butyric acid ethyl ester (Et-β-HBT-CA) was synthesized using the procedure described in Example 1 B starting with 79.68g (0.4mole) (2-Cyano-acetoxy)-butyric acid ethyl ester (Et-β-HBT-CAc), 13.2g (0.44mole) paraformaldehyde and 0.12ml piperidine. Following the catalyst removal using 16g of MP-SO₃H resin in a packed column, the oligomer was depolymerized. Crude product was re-distilled to afford 20g of colorless monomeric 3-(2-Cyano-acryloyloxy)-butyric acid ethyl ester. Final yield was 24% and the purity was 95% (by GC-MS). No polymerization along the distillation path was observed.
¹H NMR (CDCl₃, δppm): 7.03(s, 1H), 6.60(s, 1H), 5.40(m, 1H), 4.15(q, 2H), 2.65(m, 2H), 1.40(d, 3H), 1.24(t, 3H).
Boiling point: 90-98°C ~61·33 Pa

### Example 3.

### Synthesis of 2-octylcyanoacrylate

2-Octylcyanoacrylate was synthesized using the procedure described in Example 1 B starting with 98.64g (0.5mole) of 2-octylcyanoacetate, 16.5g (0.55mole) of paraformaldehyde and 0.15ml of piperidine. Following the catalyst removal using 42g of MP-SO₃H resin in a packed column, the oligomer was depolymerized. Crude product was re-distilled to afford 50g of colorless monomeric 2-octylcyanoacrylate. Final yield was 48% and the purity was 98% (by GC-MS). No polymerization along the distillation path was observed.
¹H NMR (CDCl₃, δppm): 7.02(s, 1H), 6.60(s, 1H), 5.30(m, 1H), 1.70(m, 1H), 1.58(m, 1H), 1.30(m, 11H), 0.88(t, 3H).
Boiling point: -80°C ~79·99 Pa

## Claims

1. A method for producing a cyanoacrylate monomer, comprising the steps of:
(a) reacting cyanoacetate and formaldehyde or paraformaldehyde in the presence of a base or amine base catalyst to form an α-cyanoacrylate prepolymer or oligomer mixture;
(b) subjecting the α-cyanoacrylate prepolymer or oligomer mixture to a substrate to obtain a purified α-cyanoacrylate prepolymer or oligomer by removing the catalyst; wherein, either (i) the catalyst is a base catalyst other than an amine base catalyst and said substrate is an acidic ion exchange resin, or (ii) said catalyst is an amine base catalyst and said substrate is an acidic ion exchange resin or a resin bearing other amine reacting functional groups selected from anhydrides, acid halides, aldehydes, ketones and isocyanates;
(c) subjecting the purified α-cyanoacrylate prepolymer or oligomer to depolymerization to obtain a α-cyanoacrylate monomer.

2. The method of claim 1, where the α-cyanoacrylate is selected from the group consisting of alkyl cyanoacrylates, alkoxyalkyl cyanoacrylates, alkoxycarboalkyl cyanoacrylates and combination thereof.

3. The method of claim 1, where the base catalyst is an alkali metal or alkaline earth hydroxide or a carbonate or bicarbonate

4. The method of claim 3, where the base catalyst is selected from the group consisting of sodium or potassium hydroxide, sodium or potassium methoxide, sodium or potassium ethoxide, sodium or potassium t-butoxide, sodium or potassium carbonate and sodium or potassium bicarbonate.

5. The method of claim 1, where the amine base catalyst is a primary, secondary and/or tertiary amines.

6. The method of claim 5, where the amine base catalyst is selected from the group consisting of alkylamine, dialkylamine, trialkylamine, cyclic amines, piperidine, aromatic amines and hydroxylamines.

7. The method of claim 3, where the substrate is a cationic ion exchange resin selected from the group consisting of resins having sulfonic acid or carboxylic acid groups.

8. The method of claim 4, where the substrate is a cationic ion exchange resin selected from the group consisting of resins having sulfonic acid or carboxylic acid groups.

## Patentansprüche

1. Verfahren zur Herstellung eines Cyanoacrylatmonomers, bei dem man:
(a) Cyanoacetat und Formaldehyd oder Paraformaldehyd in Gegenwart eines Basen- oder Aminbasenkatalysators zu einem α-Cyanoacrylatprepolymer-oder α-Cyanoacrylatoligomer-Gemisch umsetzt;
(b) das α-Cyanoacrylatprepolymer- oder α-Cyanoacrylatoligomer-Gemisch einem Substrat unterwirft, wobei man durch Entfernung des Katalysators ein gereinigtes α-Cyanoacrylatprepolymer oder α-Cyanoacrylatoligomer erhält; wobei entweder (i) der Katalysator ein von einem Aminbasenkatalysator verschiedener Basenkatalysator ist und das Substrat ein saures Ionenaustauscherharz ist oder (ii) der Katalysator ein Aminbasenkatalysator ist und das Substrat ein saures Ionenaustauscherharz oder ein Harz mit anderen mit Amin reagierenden funktionellen Gruppen, die unter Anhydriden, Säurehalogeniden, Aldehyden, Ketonen und Isocyanaten ausgewählt sind, ist;
(c) das gereinigte α-Cyanoacrylatprepolymer oder α-Cyanoacrylatoligomer einer Depolymerisation unterwirft, wobei man ein α-Cyanoacrylatmonomer erhält.

2. Verfahren nach Anspruch 1, bei dem man das α-Cyanoacrylat aus der Gruppe bestehend aus Alkylcyanoacrylaten, Alkoxyalkylcyanoacrylaten, Alkoxycarboalkylcyanoacrylaten und Kombinationen davon auswählt.

3. Verfahren nach Anspruch 1, bei dem es sich bei dem Basenkatalysator um ein Alkalimetall- oder Erdalkalimetallhydroxid oder ein Carbonat oder Hydrogencarbonat handelt.

4. Verfahren nach Anspruch 3, bei dem man den Basenkatalysator aus der Gruppe bestehend aus Natrium-oder Kaliumhydroxid, Natrium- oder Kaliummethoxid, Natrium- oder Kaliumethoxid, Natrium- oder Kaliumt-butoxid, Natrium- oder Kaliumcarbonat und Natrium- oder Kaliumhydrogencarbonat auswählt.

5. Verfahren nach Anspruch 1, bei dem es sich bei dem Aminbasenkatalysator um ein primäres, sekundäres und/oder tertiäres Amin handelt.

6. Verfahren nach Anspruch 5, bei dem man den Aminbasenkatalysator aus der Gruppe bestehend aus Alkylamin, Dialkylamin, Trialkylamin, cyclischen Aminen, Piperidin, aromatischen Aminen und Hydroxylaminen auswählt.

7. Verfahren nach Anspruch 3, bei dem es sich bei dem Substrat um ein kationisches Ionenaustauscherharz aus der Gruppe bestehend aus Harzen mit Sulfonsäure- oder Carbonsäuregruppen handelt.

8. Verfahren nach Anspruch 4, bei dem es sich bei dem Substrat um ein kationisches Ionenaustauscherharz aus der Gruppe bestehend aus Harzen mit Sulfonsäure- oder Carbonsäuregruppen handelt.

## Revendications

1. Procédé pour la production d'un monomère cyanoacrylate, comprenant les étapes consistant à :
(a) faire réagir un cyanoacétate et du formaldéhyde ou du paraformaldéhyde en présence d'un catalyseur basique ou basique aminé pour former un mélange de prépolymères ou oligomères d'α-cyanoacrylate ;
(b) soumettre le mélange de prépolymères ou d'oligomères d'α-cyanoacrylate à un substrat pour obtenir un prépolymère ou oligomère d'α- cyanoacrylate purifié par élimination du catalyseur ; dans lequel soit
(i) le catalyseur est un catalyseur basique autre qu'un catalyseur basique aminé et ledit substrat est une résine échangeuse d'ions acide, soit
(ii) ledit catalyseur est un catalyseur basique aminé et ledit substrat est une résine échangeuse d'ions acide ou une résine portant d'autres groupes fonctionnels réagissant avec les amines choisis parmi les anhydrides, les halogénures d'acide, des aldéhydes, les cétones et les isocyanates ;
(c) soumettre le prépolymère ou oligomère d'α- cyanoacrylate purifié à une dépolymérisation pour obtenir un monomère α-cyanoacrylate.

2. Procédé selon la revendication 1, dans lequel l'α-cyanoacrylate est choisi parmi les cyanoacrylates d'alkyle, les cyanoacrylates d'alcoxyalkyle, les cyanoacrylates d'alcoxycarboalkyle et les combinaisons de ceux-ci.

3. Procédé selon la revendication 1, dans lequel le catalyseur basique est un hydroxyde ou un carbonate ou bicarbonate de métal alcalin ou alcalinoterreux.

4. Procédé selon la revendication 3, dans lequel le catalyseur basique est choisi parmi l'hydroxyde de sodium ou de potassium, le méthylate de sodium ou de potassium, l'éthylate de sodium ou de potassium, le t-butylate de sodium ou de potassium, le carbonate de sodium ou de potassium et le bicarbonate de sodium ou de potassium.

5. Procédé selon la revendication 1, dans lequel le catalyseur basique aminé est une amine primaire, secondaire et/ou tertiaire.

6. Procédé selon la revendication 5, dans lequel le catalyseur basique aminé est choisi parmi une alkylamine, une dialkylamine, une trialkylamine, les amines cycliques, la pipéridine, les amines aromatiques et les hydroxylamines.

7. Procédé selon la revendication 3, dans lequel le substrat est une résine échangeuse d'ions cationique choisie parmi les résines ayant des groupes acides sulfoniques ou acides carboxyliques.

8. Procédé selon la revendication 4, dans lequel le substrat est une résine échangeuse d'ions cationique choisie parmi les résines ayant des groupes acides sulfoniques ou acides carboxyliques.
